# EUROPEAN PATENT SPECIFICATION

(11) **EP 0 415 010 B1**
(45) Date of publication and mention of the grant of the patent: **18.01.1995**
(21) Application number: 90110917.3
(22) Date of filing: 08.06.1990
(51) Int. Cl.: A61B 7/04, A61B 5/0205

(54) **Diagnostic apparatus for functional examination of circulatory organ system**
Diagnostikgerät zur funktionellen Untersuchung des Kreislaufsystems
Appareil de diagnostique pour l'examen fonctionnel du système circulatoire

(30) Priority: 31.08.1989 JP 223347/89
(43) Date of publication of application: 06.03.1991
(73) Proprietor: SONY CORPORATION, Tokyo (JP)
(72) Inventor: Baek Kwang Chul, Seoul (KR)
(74) Representative: Strehl Schübel-Hopf Groening & Partner

(56) References cited:
- US-A- 4 066 066
- US-A- 4 396 018
- US-A- 4 586 514
- IEEE TRANSACTIONS ON BIO-MEDICAL ENGINEERING. vol. BME30, no. 6, June 1983, NEWYORK US pages 348 - 352; LEE et al.: "Spectrum analysis of human pulse."

## Description

### Background of the Invention

### 〈Field of the Invention〉

The present invention relates to a diagnostic apparatus for a functional examination of a circulatory organ system, and more particulary to a pulse diagnostic apparatus applicable to Chinese medicine, an electrocardiograph and the like.

### 〈Description of the Prior Art〉

As a diagnostic apparatus for executing a functional examination of circulatory organs of a human body, a sphygmomanometer, an electrocardiograph, a plethysmographic apparatus and the like are provided in Occidental medicine, and a pulse diagnostic record apparatus relating to U.S.P. No. 4,066,066 is familiar in Chinese medicine.

The above apparatuses have a point of sameness in that a physiological variation of circulatory organs is visibly expressed by using electronic apparatuses. Namely, an electrocardiograph, a plethysmographic apparatus, a pulse diagnostic record apparatus and the like record natural signals occurring in the human body, specifically in a circulatory organ system through a series of amplifying circuits. The shapes of so-recorded graphs are analyzed and such graphs are used as materials for grasping the state of the human body.

An electrocardiograph records the potential difference generated by the pulse of heart and judges the function of the heart. However, the electrocardiogram has the drawbacks that, since it is used in a clinical examination and in considerable cases the judgement of heart disease is not made by the results, the reading of electrocardiogram is required to be made together with clinical observations.

On the other hand, a plethysmographic is new research. It records a plethysmographic on traditional factors, velocity and acceleration together with especially an amount of blood flow moving along capillaries in order to exceed the limit of diagnoses which an electrocardiogram has, and it becomes possible to diagnose several diseases, especially the diseases of adult people (see U.S.A. Patent No. 3,881,481; 3,920,004; 4,154238 and 4,432,374).

Moreover, a pulse diagnostic record apparatus of the above-mentioned U.S.P. No. 4,066,066 records the wavy pattern of vibrations which is sensed by piezoelectric microphones and obtained in a circuit utilized for an existing plethysmogram. The diagnosis of various diseases was made possible by analyzing the so-obtained record corresponding to the eastern medicine system.

As described above, the plethysmographic and the pulse diagnostic record are identical in so far as they are a means for diagnosis of diseases in the circulatory organ system.

Since the wavy patterns of graph to be obtained by, for example, not only the above-mentioned pulse diagnostic record apparatus but also the existing electrocardiograph have complex forms, the operators have to gain experience to give the appropriate judgement by taking a look at these graphs. Additionally, the operators are forced to give a subjective judgement. As a result, this kind of apparatus according to the prior art is not sufficiently satisfactory for wide usability.

IEEE Transactions on Biomedical Engineering, volume BME-30, 1983, No 6, pp. 348-352 discloses a method for analyzing signals obtained by pulse diagnosis. However this method involves a Fourier Transform of the signals and therefore requires a powerful computer.

### Summary of the Invention

Taking into consideration the above-mentioned prior art, an object of the present invention is to provide a diagnostic apparatus for a functional examination of a circulatory organ system that displays specifically and visibly characteristics of examined diseases.

The above-mentioned object is achieved by the apparatus of claim 1. The points where the microphones are provided are three (3) points of the pulsing points near the wrist consisting of Point 1 (sun), Point 2 (seki), Point 3 (shaku) which are stylus portions of an antebrachial bone of arms.

According to the present invention of the above-mentioned constitution, the wavy patterns of pulses detected by the sensor portion are filtered by the filter portion having the frequency band substantially from 14Hz to 37Hz. As a result, the so-obtained wavy patterns become ones in which the characteristics necessary for the examination are emphasized.

### Brief Description of the Drawings

Fig.1 is a block diagram showing an embodiment of the present invention;
Fig.2 is a detailed circuit diagram;
Fig.3 is an explanatory picture showing conceptually the extracted meter;
Fig.4 is a frequency characteristic graph;
Fig.5(a) to Fig.7(a) are wavy patterns graphs showing the wavy patterns before they are filtered; and
Fig.5(b) to Fig.7(b) are wavy patterns graphs showing the wavy patterns after they are filtered.

### Detailed Description of the Preferred Embodiments

Hereinafter, a preferred embodiment of the present invention will be explained by using the accompanying drawings.

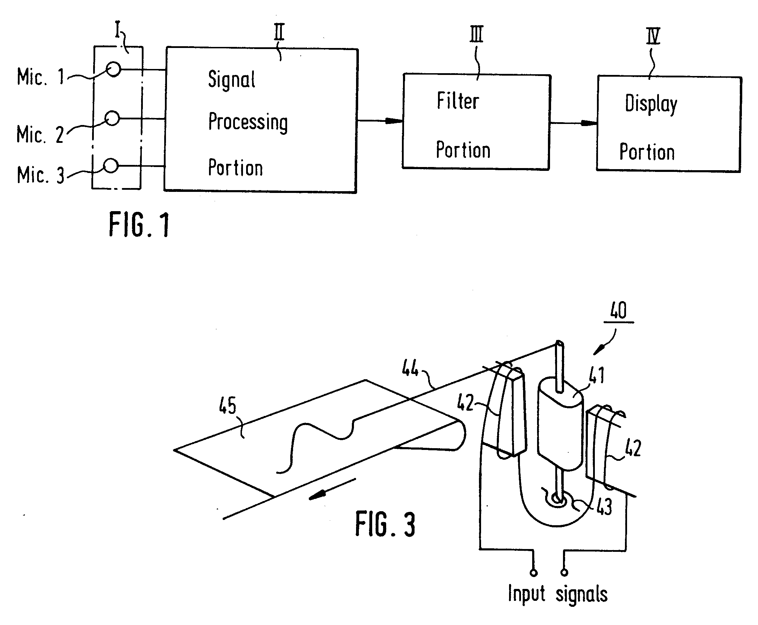

As shown in Fig.1, a preferred embodiment is a pulse diagnostic apparatus for Chinese medicine which adds a filter portion and a display portion to the same sensor portion and signal process portion as that in U.S.P. No. 4,066,066. A sensor I is formed by crystal microphones Mic.1, Mic.2, Mic.3 composed of piezoelectric elements, each of which is releasably provided on one point of Point 1 (sun), Point 2 (seki), Point 3 (shaku) of the pulsing points. A signal process portion II amplifies output signals of each of the microphones Mic.1 to Mic.3 and in addition executes the signal process of noise reduction and the like. A filter portion III filters frequency components in the vicinity of 14Hz to 37Hz of the output signals of the signal process portion II and the output signals filtered by the filter portion III are visible at a display portion IV.

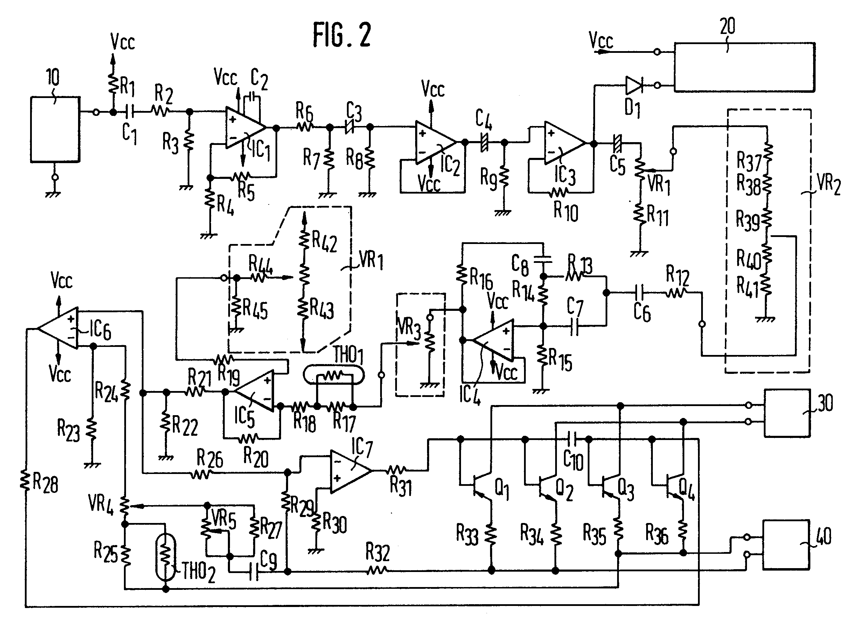

Fig.2 is a circuit diagram showing a detailed composition of the above-mentioned embodiment. The figure shows a signal process system. However, a pulse diagnostic apparatus in this embodiment has three (3) signal process systems in parallel one of which is the same as that shown in Fig.2 and each signal process system processes three (3) kinds of pulse waves of Point 1 (sun), Point 2 (seki), Point 3 (shaku). As shown in Fig.2, the output of a sensor 10 which is a crystal microphone is inputted into an OP amplifier IC₁, through a capacitor C₁, and a resistance R₂ The signals amplified by the OP amplifier IC₁ undergo noise suppression by resistances R₆, R₇, R₈ and a capacitor C₃, and they are inputted into an OP amplifier IC₂. The signals amplified by the OP amplifier IC₂ are inputted into an OP amplifier IC₃ through a capacitor C₄ and a resistance R₉. The signals amplified by the OP amplifier IC₃ are inputted into a level display 20 and the amplitude is visibly displayed at the level display 20, and additionally they are inputted into a variable resistance VR₁ grounded by a resistance R₁₁ through a capacitor C₅. A needle of the variable resistance VR₁ is connected to an external variable resistance VR₂ whose one end is grounded. On the other hand, a needle of the external variable resistance VR₂ is inputted into an OP amplifier IC₄ through a resistance R₁₄, capacitors C₆, C₇, and resistances R₁₃, R₁₄. The output of the OP amplifier IC₄ is inputted into an OP amplifier IC₅ through a variable resistance VR₃ and resistances R₁₇, R₁₈. In this portion, a temperature compensation thyristor TH₀₁ is connected in parallel to the resistance R₁₇.

The signals amplified by the OP amplifier IC₅ are respectively supplied to OP amplifiers IC₆, IC₇ through a resistance R₂₁. The output side of the OP amplifiers IC₆, IC₇ are connected to each other through a capacitor C₁₀ and are respectively connected to the bases of transistors Q₁, Q₂ Q₃_{,} Q₄. A relay 30 is supplied the electric power with transistors Q₁ to Q₄. A power amplifier portion of push-pull style comprises OP amplifiers IC₆, IC₇ and transistors Q₁ to Q₄. Since a galvanometer is used as a meter 40 in the embodiment, the above-mentioned power amplifier portion is provided to drive the meter 40. If an oscilloscope is used as the display portion IV, the above-mentioned power amplifier portion is unnecessary.

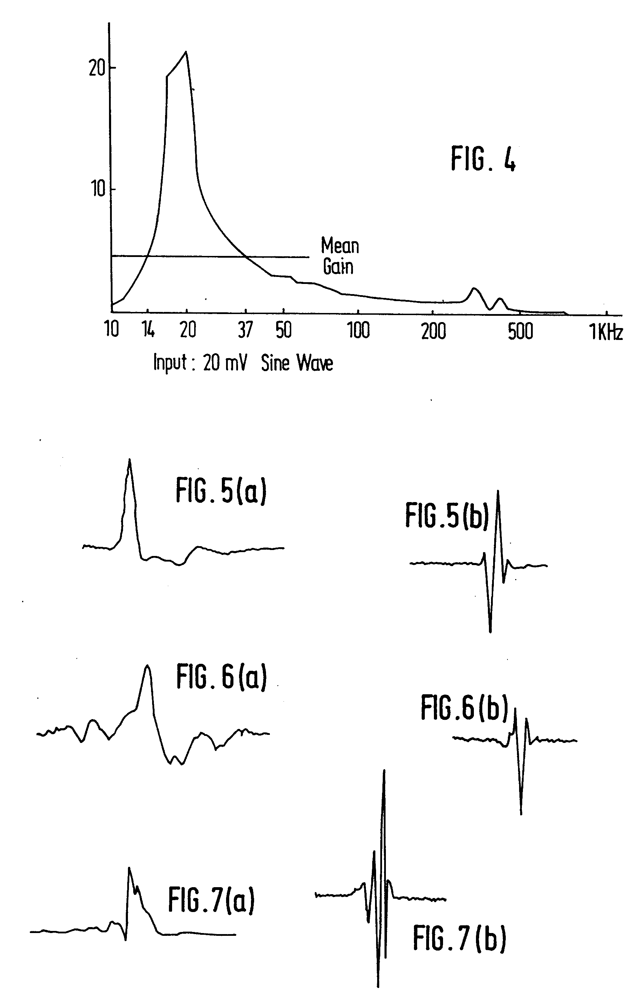

The meter 40 in the embodiment is formed by a galvanometer, and the filter portion III and the display portion IV are united. Namely, the meter 40, as shown in Fig.3 by extracting such portion, comprises a magnet 41 of a movable portion, a magnet coil 42 of a fixed portion which interposes the magnet 41 and which is symmetrically disposed, a spring 43, a pen 44 and a graph paper 45. An amount of rotary movement of the magnet 41 alters corresponding to the amplitude of output signals from the power amplifier portion which are supplied to the magnet coil 42, and the rotary movement is visibly displayed by drawing the amplitude corresponding to the rotary movement on the graph paper 45 with the pen 44.

An amount of rotary movement of the magnet 41 becomes zero in such a state that an electromagnetic force between the magnet 41 and the magnet coil 42, and a biasing force of the spring 43 are balanced. A resonance frequency of a mechanical vibration system of the meter 40 alters by adjusting a spring constant of the spring 43. Accordingly, a desired frequency characteristic is set by adjusting a spring constant of the spring 43.

A frequency characteristic of the meter 40 in the case of this embodiment is shown in Fig.4. As shown in Fig.4, the meter 40 is recognized to be a band filter from 14Hz to 37Hz.

In this embodiment, the signals indicating a normal pulse which is the output of the signal process portion II as shown in Fig.5(a) are visibly displayed as a wavy pattern as shown in Fig.5(b) on the graph paper 45 of the display portion IV. Hereinafter, as described above, a negative pulse shown in Fig.6(a) is visibly displayed as a wavy patter as shown in Fig.6(b) and a positive pulse shown in Fig.7(a) is visibly displayed as a wavy pattern as shown in Fig.7(b). If referring to Fig.5(b) to Fig.7(b), since the wavy patterns are more emphasized in their distinctive parts than those shown in Fig.5(a) to Fig.7(a), they are recognized to be easy to read.

Moreover, in the above-mentioned embodiment, the filter portion III is formed by the mechanical vibration system of the meter 40. However, an electric filter having the same frequency band characteristic may be used, too. In the case of the electric filter, an oscilloscope is suitable for the display portion IV into which the output signals of the OP amplifier IC₅ in a circuit as shown in Fig.2 are inputted and in which the output signals of such filter is changed to visible waves. Moreover, the present invention is applicable to not only a pulse diagnostic apparatus, but also devices which perform a functional examination of a circulatory organ system, for example an electrocardiograph and the like.

As concretely described above showing an embodiment, since the wavy patterns detected by a sensor portion are visibly displayed by being filtered at a filter portion according to the present invention, a specific examination may be performed easily and objectively by observing the visible wavy patterns.

## Claims

1. A diagnostic apparatus for functional examination of a circulatory organ system of a human body, comprising:
a sensor device (I) including three microphones each for generating a pulsing point signal when being placed on one of three pulsing points near a wrist,
a signal processing device (II) for receiving and amplifying said pulsing point signals from said microphones (10), and
a display device (IV, 40) coupled to said signal processing device for generating a visible graph of said pulsing point signals,
characterised by a filter device (III) including a band pass filter for filtering the pulsing point signals and selecting components of the pulsing point signals substantially from 14 Hz to 37 Hz for display.

2. An apparatus according to claim 1, characterised in that said display device comprises a galvanometer (40) and said filter device (III) is provided by a mechanical resonance system of the galvanometer.

3. An apparatus according to claim 1, characterised in that said filter device (III) is an electric filter.

## Patentansprüche

1. Diagnosevorrichtung zur funktionellen Überprüfung eines Kreislaufsystems eines menschlichen Körpers, aufweisend:
eine Sensoreinrichtung (I) mit drei Mikrophonen, um jeweils bei Anordnung auf einem von drei Pulspunkten in der Nähe eines Handgelenks ein Pulspunktsignal zu erzeugen,
eine Signalverarbeitungseinrichtung (II) zum Empfang und zur Verstärkung der Pulspunktsignale der Mikrophone (10), und
eine Anzeigeeinrichtung (IV, 40), die mit der Signalverarbeitungseinrichtung verbunden ist, um eine sichtbare, graphische Darstellung der Pulspunktsignale zu erzeugen,
gekennzeichnet durch eine Filtereinrichtung (III) mit einem Bandpassfilter zum Filtern der Pulspunktsignale und zur Auswahl von Komponenten der Pulspunktsignale von im wesentlichen 14 Hz bis 37 Hz zur Anzeige.

2. Vorrichtung nach Anspruch 1, dadurch gekennzeichnet, daß die Anzeigeeinrichtung ein Galvanometer (40) beinhaltet und daß die Filtereinrichtung (III) von einem mechanischen Resonanzsystem des Galvanometers gebildet ist.

3. Vorrichtung nach Anspruch 1, dadurch gekennzeichnet, daß die Filtereinrichtung (III) ein elektrisches Filter ist.

## Revendications

1. Appareil de diagnostic pour l'examen fonctionnel de l'appareil circulatoire du corps humain, comprenant:
un dispositif de détection (I) comportant trois microphones servant chacun à produire un signal de point de pulsations quand on le place sur un des trois points de pulsations près du poignet,
un dispositif (II) de traitement de signaux pour recevoir et amplifier lesdits signaux de points de pulsations provenant desdits microphones (10), et
un dispositif de visualisation (IV, 40) couplé audit dispositif de traitement de signaux pour produire un graphique visible desdits signaux de points de pulsations,
caractérisé par un dispositif de filtrage (III) comportant un filtre passe-bande pour filtrer les signaux de points de pulsations et sélectionner des composantes des signaux de points de pulsations, sensiblement comprises entre 14 Hz et 37 Hz, pour l'affichage.

2. Appareil selon la revendication 1, caractérisé en ce que ledit dispositif de visualisation comporte un galvanomètre (40) et ledit dispositif de filtrage (III) est constitué par un système mécanique à résonance du galvanomètre.

3. Appareil selon la revendication 1, caractérisé en ce que ledit dispositif de filtrage (III) est un filtre électrique.
